# FASCICULE DE BREVET EUROPEEN

(11) **EP 0 926 149 B1**
(45) Date de publication et mention de la délivrance du brevet: **07.03.2001**
(21) Numéro de dépôt: 98402823.3
(22) Date de dépôt: 13.11.1998
(51) Int. Cl.: C07D 487/04, A61K 7/13

(54) **Compositions de teinture des fibres kératinques contenant des 3-aminopyrazolo-[1,5-a]-pyrimidines, procédé de teinture, 3-aminopyrazolo-[1,5-a]-pyrimidines et leur procédé de préparation**
Zusammensetzungen zum Färbung von keratinhaltigen Fasern enthaltend 3-Aminopyrazolo-(1,5-a)-pyrimidine, Färbeverfahren, 3-Aminopyrazolo-(1,5-a)-pyrimidine und ihre Herstellung
Keratin fibre dye composition containing 3-aminopyrazolo-(1,5-a)-pyrimidines, dyeing method , 3-aminopyrazolo-(1,5-a)-pyrimidines and their preparation

(30) Priorité: 03.12.1997 FR 9715244
(43) Date de publication de la demande: 30.06.1999
(73) Titulaire: L'OREAL, 75008 Paris (FR)
(72) Inventeur: Terranova, Eric, 92270 Bois Colombes (FR); Fadli, Aziz, 77500 Chelles (FR); Lagrange, Alain, 77770 Coupvray (FR)
(74) Mandataire: Goulard, Sophie

(56) Documents cités:
- EP-A- 0 433 854
- EP-A- 0 433 855
- WO-A-97/49378
- DE-A- 2 257 547
- DE-A- 2 920 537
- DE-A- 4 029 324
- DE-A- 4 133 957

## Description

L'invention a pour objet de nouvelles compositions pour la teinture d'oxydation des fibres kératiniques comprenant au moins une 3-amino pyrazolo-[1,5-a]-pyrimidine à titre de base d'oxydation, le procédé de teinture mettant en oeuvre cette composition, de nouvelles 3-amino pyrazolo-[1,5-a]-pyrimidines ainsi que leur procédé de préparation.

Il est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols, des composés hétérocycliques tels que des dérivés de diaminopyrazole, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants. Ces composés ont pour point commun de posséder un groupement amino et un groupement hydroxyle ou deux groupements amino, ce qui leur confère leur caractère de base d'oxydation.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée, présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

Il a déjà été proposé, notamment dans la demande de brevet DE 4 029 324, d'utiliser certaines 2-hydroxy pyrazolo-[1,5-a]-pyrimidines, pouvant être substitués par des radicaux alkyles en C₁-C₄ en position 4, 5 et/ou 6, comme coupleurs pour la teinture d'oxydation des fibres kératiniques.

Il a été proposé aussi dans la demande de brevet DE 4 133 957, d'utiliser certains dérivés de pyrazolo-[1,5-a]-pyrimidine appartenant à la famille des tétrahydro pyrazolo-[1,5-a]-pyrimidine comme précurseurs de colorant d'oxydation pour la teinture d'oxydation des fibres kératiniques. La demande de brevet WO-9-97/49378 propose également d'utiliser certaines diamino pyrazolo[1,5-a]pyrimidines ou certaines aminohydroxy pyrazolo[1,5-a]pyrimidines comme précurseurs de colorant d'oxydation pour la teinture d'oxydation des fibres kératiniques. La demanderesse vient maintenant de découvrir, de façon totalement inattendue et surprenante, une nouvelle famille de 3-amino pyrazolo-[1,5-a]-pyrimidine définie ci-après, pour partie nouveaux en soi, pouvant convenir pour une utilisation comme base d'oxydation alors que ces composés ne comportent qu'un seul groupement amino, mais en outre permettant d'obtenir des compositions tinctoriales qui conduisent à des colorations puissantes et qui présentent une bonne tenue face aux agents extérieurs (lumière, intempéries, lavage, ondulation permanente, transpiration, frottements). Enfin, ces composés s'avèrent être aisément synthétisables.

Ces découvertes sont à la base de la présente invention.

L'invention a donc pour premier objet une composition pour la teinture d'oxydation des fibres kératiniques et en particulier des fibres kératiniques humaines telles que les cheveux, caractérisée par le fait qu'elle comprend, dans un milieu approprié pour la teinture, au moins un coupleur et au moins une 3-amino pyrazolo-[1,5-a]-pyrimidine à titre de base d'oxydation et/ou un de ses sels d'addition avec un acide ou avec une base choisie parmi :
- la pyrazolo-[1,5-a]-pyrimidin-3-ylamine ;
- la 5,6,7-triméthyl pyrazolo-[1,5-a]-pyrimidin-3-ylamine ;
- la 7-méthyl pyrazolo-[1,5-a]-pyrimidin-3-ylamine ;
- la 5-méthyl pyrazolo-[1,5-a]-pyrimidin-3-ylamine ;
- la 7-méthoxy pyrazolof[1,5-a]-pyrimidin-3-ylamine ;
- la 5-méthoxy pyrazolo-[1,5-a]-pyrimidin-3-ylamine ;
- la 7-méthoxy 5-méthyl pyrazolo-[1,5-a]-pyrimidin-3-ylamine ;
- la 2,5,6,7-tetraméthyl pyrazolo-[1,5-a]-pyrimidin-3-ylamine ;
- la 2-méthoxy 5,7-diméthyl pyrazolo-[1,5-a]-pyrimidin-3-ylamine ;
- la 5,7-di-tert-butyl pyrazolo-[1,5-a]-pyrimidin-3-ylamine ;
- la 5,7-di-trifluorométhyl pyrazolo-[1,5-a]-pyrimidin-3-ylamine ; .
- la 2,6-diméthyl pyrazolo-[1,5-a]-pyrimidin-3-ylamine ;
- la 2-chloro 5,7-diméthyl pyrazolo-[1,5-a]-pyrimidin-3-ylamine ;
- la 7-méthyl 2-tert-butyl 5-trifluorométhyl pyrazolo-[1,5-a]-pyrimidin-3-ylamine ;
et leurs sels d'addition avec un acide ou avec une base.

Parmi les 3-amino pyrazolo-[1,5-a]-pyrimidines utilisables à titre de base d'oxydation dans les compositions conformes à l'invention, on préfère tout particulièrement :
- la pyrazolo-[1,5-a]-pyrimidin-3-ylamine ;
- la 5,6,7-triméthyl pyrazolo-[1,5-a]-pyrimidin-3-ylamine ;
- la 2,5,6,7-tetraméthyl pyrazolo-[1,5-a]-pyrimidin-3-ylamine ;
- la 2-méthoxy 5,7-diméthyl pyrazolo-[1,5-a]-pyrimidin-3-ylamine ;
- la 5,7-di-trifluorométhyl pyrazolo-[1,5-a]-pyrimidin-3-ylamine ;
- la 7-méthyl pyrazolo-[1,5-a]-pyrimidin-3-ylamine ;
- la 5-méthyl pyrazolo-[1,5-a]-pyrimidin-3-ylamine ;
et leurs sels d'addition avec un acide ou avec une base.

Pour leur grande majorité, les 3-amino pyrazolo-[1,5-a]-pyrimidines sont des composés connus dans le domaine pharmaceutique, et sont décrites notamment dans les demandes de brevet DE 2 257 547, EP-A-0 433 854, et EP-A-0 433 855.

La ou les 3-amino pyrazolo-[1,5-a]-pyrimidines ci-dessus représentent de préférence de 0,0005 à 12 % en poids environ du poids total de la composition tinctoriale, et encore plus préférentiellement de 0,005 à 6 % en poids environ de ce poids.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; le glycérol ; les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (II) suivante : 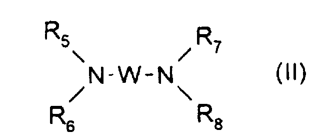 dans laquelle W est un reste propylène éventuellement substitué par un groupement hydroxyle ou un radical alkyle en C₁-C₄ ; R₅, R₆, R₇ et R₈, identiques ou différents, représentent un atome d'hydrogène, un radical alkyle en C₁-C₄ ou hydroxyalkyle en C₁-C₄.

Selon une forme de réalisation préférée, la composition de teinture d'oxydation conforme à l'invention renferme en outre un ou plusieurs coupleurs de façon à modifier ou à enrichir en reflets les nuances obtenues en mettant en oeuvre les composés 3-aminopyrazolo-[1,5-a]pyrimidines.

Les coupleurs utilisables dans les compositions de teinture d'oxydation conformes à l'invention peuvent être choisis parmi les coupleurs utilisés de façon classique en teinture d'oxydation et parmi lesquels on peut notamment citer les métaphénylènediamines, les méta-aminophénols, les métadiphénols et les coupleurs hétérocycliques.

Ces coupleurs sont plus particulièrement choisis parmi le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxy benzène, le 1,3-diamino benzène, le 1,3-bis-(2,4-diaminophénoxy) propane, le sésamol, l'α-naphtol, le 6-hydroxy indole, le 4-hydroxy indole, le 4-hydroxy N-méthyl indole, la 6-hydroxy indoline, la 2,6-dihydroxy 4-méthyl pyridine, le 1-H 3-méthyl pyrazole 5-one, le 1-phényl 3-méthyl pyrazole 5-one, et leurs sels d'addition.

Le ou les coupleurs représentent de préférence de 0,0001 à 10 % en poids environ du poids total de la composition tinctoriale et encore plus préférentiellement de 0,005 à 5 % en poids environ de ce poids.

La composition tinctoriale conforme à l'invention peut encore contenir, en plus des colorants définis ci-dessus, au moins une base d'oxydation additionnelle qui peut être choisie parmi les bases d'oxydation classiquement utilisées en teinture d'oxydation et parmi lesquelles on peut notamment citer les paraphénylènediamines, les bis-phénylalkylènediamines, les paraaminophénols, les ortho-aminophénols et des bases hétérocycliques différentes des 3-amino pyrazolo-[1,5-a]-pyrimidines utilisées conformément à l'invention.

Parmi les paraphénylènediamines, on peut plus particulièrement citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, et leurs sels d'addition avec un acide.

Parmi les paraphénylènediamines citées ci-dessus, on préfère tout particulièrement la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide.

Parmi les bis-phénylalkylènediamines, on peut plus particulièrement citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diaminophénoxy)-3,5-dioxaoctane, et leurs sels d'addition avec un acide.

Parmi les para-aminophénols, on peut plus particulièrement citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le (4-amino-2-aminométhyl) phénol, le 4-amino 2-fluoro phénol; et leurs sels d'addition avec un acide.

Parmi les ortho-aminophénols, on peut plus particulièrement citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut plus particulièrement citer à titre d'exemple, les dérivés pyridiniques, les dérivés pyrimidiniques et les dérivés pyrazoliques.

Lorsqu'elles sont utilisées, la ou les bases d'oxydation additionnelles représentent de préférence de 0,0005 à 12 % en poids environ du poids total de la composition tinctoriale, et encore plus préférentiellement de 0,005 à 6 % en poids environ de ce poids.

D'une manière générale, les sels d'addition avec un acide utilisables dans le cadre des compositions tinctoriales de l'invention (composés 3-aminopyrazolo-[1,5-a]pyrimidine, bases d'oxydation additionnelles et coupleurs) sont notamment choisis parmi les chlorhydrates, les bromhydrates, les sulfates, les citrates, les succinates, les tartrates, les lactates et les acétates. Les sels d'addition avec une base utilisables dans le cadre des compositions tinctoriales de l'invention (composés 3-aminopyrazolo-[1,5-a]pyrimidine, bases d'oxydation additionnelles et coupleurs) sont notamment ceux obtenus avec la soude, la potasse, l'ammoniaque ou les amines.

La composition tinctoriale conforme à l'invention peut en outre renfermer un ou plusieurs colorants directs pouvant notamment être choisis parmi les colorants nitrés de la série benzéniques.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

L'invention a également pour objet un procédé de teinture des fibres kératiniques et en particulier des fibres kératiniques humaines telles que les cheveux mettant en oeuvre la composition tinctoriale telle que définie précédemment.

Selon ce procédé, on applique sur les fibres au moins une composition tinctoriale telle que définie précédemment, pendant un temps suffisant pour développer la coloration désirée, soit à l'air, soit à l'aide d'un agent oxydant. La composition tinctoriale peut éventuellement contenir des catalyseurs d'oxydation, afin d'accélérer le processus d'oxydation.

Selon une première forme de mise en oeuvre du procédé de l'invention, la coloration des fibres peut être effectuée sans addition d'un agent oxydant, au seul contact de l'oxygène de l'air.

Selon une deuxième forme de mise en oeuvre du procédé de l'invention, on applique sur les fibres au moins une composition tinctoriale telle que définie précédemment, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

Selon cette deuxième forme de mise en oeuvre du procédé de teinture de l'invention, on mélange de préférence, au moment de l'emploi, la composition tinctoriale décrite ci-dessus avec une composition oxydante contenant, dans un milieu approprié pour la teinture, au moins un agent oxydant présent en une quantité suffisante pour développer une coloration. Le mélange obtenu est ensuite appliqué sur les fibres kératiniques et on laisse poser pendant 3 à 50 minutes environ, de préférence 5 à 30 minutes environ, après quoi on rince, on lave au shampooing, on rince à nouveau et on sèche.

L'agent oxydant présent dans la composition oxydante telle que définie ci-dessus peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les persels tels que les perborates et persulfates et les enzymes telles que les peroxydases et les oxydo-réductases à 2 électrons. Le peroxyde d'hydrogène est particulièrement préféré.

Le pH de la composition oxydante renfermant l'agent oxydant tel que défini ci-dessus est tel qu'après mélange avec la composition tinctoriale, le pH de la composition résultante appliquée sur les fibres kératiniques varie de préférence préférence entre 3 et 12 environ, et encore plus préférentiellement entre 5 et 11. Il est ajusté à la valeur désirée au moyen d'agents acidifiants ou alcalinisants habituellement utilisés en teinture des fibres kératiniques et tels que définis précédemment.

La composition oxydante telle que définie ci-dessus peut également renfermer divers adjuvants utilisés classiquement dans les compositions pour la teinture des cheveux et tels que définis précédemment.

La composition qui est finalement appliquée sur les fibres kératiniques peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de l'invention est un dispositif à plusieurs compartiments ou "kit" de teinture ou tout autre système de conditionnement à plusieurs compartiments dont un premier compartiment renferme la composition tinctoriale telle que définie ci-dessus et un second compartiment renferme la composition oxydante telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les composés 3-aminopyrazolo-[1,5-a]pyrimidine utilisés à titre de base d'oxydation dans le cadre de la présente invention, sont nouveaux et, à ce titre, constituent un autre objet de l'invention.

Ces nouvelles 3-amino de pyrazolo-[1,5-a]-pyrimidines, sont :
- la pyrazolo-[1,5-a]-pyrimidin-3-ylamine ;
- la 5,6,7-triméthyl pyrazolo-[1,5-a]-pyrimidin-3-ylamine ;
- la 7-méthyl pyrazolo-[1,5-a]-pyrimidin-3-ylamine ;
- la 5-méthyl pyrazolo-[1,5-a]-pyrimidin-3-ylamine ;
- la 7-méthoxy pyrazolo-[1,5-a]-pyrimidin-3-ylamine ;
- la 5-méthoxy pyrazolo-[1,5-a]-pyrimidin-3-ylamine ;
- la 7-méthoxy -5-methyl pyrazolo-[1,5-a]-pyrimidin-3-ylamine ;
- la 2,5,6,7-tetraméthyl pyrazolo-[1,5-a]-pyrimidin-3-ylamine ;
- la 5,7-di-tert-butyl pyrazolo-[1,5-a]-pyrimidin-3-ylamine ;
- la 5,7-di-trifluorométhyl pyrazolo-[1,5-a]-pyrimidin-3-ylamine ;
et leurs sels d'addition avec un acide ou avec une base.

Ces nouvelles 3-amino pyrazolo-[1,5-a]-pyrimidines peuvent être préparées selon des méthodes connues et décrites dans la littérature.

A titre d'exemple, on peut effectuer une réaction de cyclocondensation entre un dérivé de 3(5)-amino-4-nitro pyrazole et un β-cétoester, une β-dicétone ou un β-cétoaldéhyde pour former la structure pyrazolo-[1,5-a]-pyrimidine. Cette réaction peut s'effectuer en s'inspirant des méthodes décrites dans les références suivantes :
- EP-A-628559 BEIERSDORF-LILLY
- G. Mühmel, R. Hanke, E. Breitmaier, Synthesis, 673, 1982..

Le dérivé nitré obtenu peut ensuite être réduit pour conduire à la 3-amino pyrazolo-[1,5-a]-pyrimidine attendue selon des procédés connus (R. Hemmer, W. Lürken, dans Houben-Weyl, "Methoden der Organischen Chemie", vol.E16d, p 815ff.). On préférera utiliser des métaux comme le palladium (Pd), le platine (Pt) ou le nickel (Ni) en présence de donneur d'hydrogène comme le formiate d'ammonium, l'acide formique ou encore le cyclohexène à la place de l'hydrogène (S. Ram, R.E. Ehrenkaufer, Synthesis, 91, 1988). On pourra également utiliser des métaux comme le zinc (Zn), l'étain (Sn) ou le fer (Fe) en milieu acide tel que l'acide chlorhydrique aqueux ou l'acide acétique aqueux, éventuellement avec addition d'un solvant organique comme le méthanol, l'éthanol ou le tétrahydrofurane.

Ou bien, on peut effectuer une réaction de cyclocondensation entre un dérivé de 3(5)-amino pyrazole et un β-cétoester, une β-dicétone ou un β-cétoaldéhyde pour former la structure pyrazolo-[1,5-a]-pyrimidine. Cette réaction peut s'effectuer en s'inspirant des méthodes décrites dans les références suivantes:
- R.H. Springer, M.B. Scholten, D.E. O'Brien, T. Novinson, J.P. Miller, R.K. Robins, J. Med. Chem., 25, 235, 1982.
- T. Novinson, R.K. Robins, T.R. Matthews, J. Med. Chem., 20, 386, 1977.
- K. Nagahara, H. Kawano, S. Sasaoka, C. Ukawa, T.Hirama, A. Takada, J. Heterocyclic Chemistry, 239, 1994.

On peut ensuite procéder à une réaction de nitration de cette structure pyrazolo-[1,5-a]-pyrimidine selon des méthodes bien connues. A titre d'exemple, on pourra se reporter à la référence suivante :
- K. Senga, T. Novinson, R.H. Springer, R.P. Rao, D.E. O'Brien, R.K. Robins, H.R. Wilson, J. Med. Chem., 18(3), 312, 1975.

Le dérivé nitré peut ensuite être réduit comme ci-dessus pour conduire à la 3-amino pyrazolo-[1,5-a]-pyrimidine attendue.

Les 3-amino pyrazolo-[1,5]-pyrimidines, ainsi que leurs sels d'addition telles que définies ci-dessus peuvent également être utilisées comme base d'oxydation dans et pour la préparation de compositions destinées à la photographie ou à l'imagerie chimique.

Les exemples qui suivent sont destinés à illustrer l'invention sans pour autant en limiter la portée.

### EXEMPLES DE SYNTHESE

### EXEMPLE 1: Synthèse du chlorhydrate de pyrazolo-[1,5-a]pyrimidin-3-ylamine

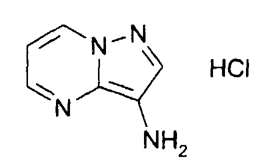

### a) Préparation de la 3-nitro-pyrazolo-[1,5-a]-pyrimidine

Dans un ballon tricol de 500 ml équipé d'une agitation magnétique, d'un réfrigérant et d'un thermomètre, on a introduit 44 g de malonaldéhyde bis-diéthylacétal, 300 cc d'acide acétique et 30 g de chlorhydrate de 4-nitro-2H-pyrazol-3-ylamine (préparé selon H. Dorn et H. Dilcher, Liebigs Ann.Chem.,707, 141, 1967). On a porté le milieu au reflux pendant 5 heures, puis évaporé environ 100 cc d'acide acétique. On a filtré le solide et on l'a rincé à l'éther diisopropylique. On a obtenu 27,7 g de 3-nitro-pyrazolo-[1,5-a]-pyrimidine après séchage sous vide et sur anhydride phosphorique à 40°C (Rendement = 92,5%)
- RMN 1H (DMSO d6; 200MHz) :: 3,34 (s; 1H) ; 7,51 (dxd ; J1 = 4,3 Hz et J2 = 6,9 Hz ; 1H) ; 9,04 (dxd ; J1 = 4,3 Hz et J3 = 1,2 Hz ; 1H) ; 9,09 (s ; 1H) ; 9,44 (dxd ; J2 = 6,9 Hz et J3 = 1,2 Hz ; 1H)

### b) Préparation du chlorhydrate de pyrazolo-[1,5-a]-pyrimidin-3-ylamine

Dans un ballon tricol de 250 ml équipé d'une agitation magnétique, d'un réfrigérant et d'un thermomètre, on a introduit 100 cc d'éthanol, 12 cc d'eau, 3,3g de chlorure d'ammonium et 10 g de 3-nitro-pyrazolo-[1,5-a]-pyrimidine obtenue ci-dessus à l'étape précédente. On a porté le milieu au reflux. On a retiré le chauffage et ajouté 16 g de zinc en poudre par petites portions de façon à maintenir le reflux. Après complète addition, on a chauffé pendant 3 heures au reflux. On a filtré à chaud les sels de zinc. Le filtrat a été refroidi jusqu'à cristallisation. On a filtré le produit cristallisé et on l'a dissout dans 100 cc d'éthanol absolu. Après barbotage d'acide chlorhydrique gazeux à travers la solution éthanolique, on a fait précipiter le chlorhydrate par addition de 1 litre d'éther diisopropylique. On a obtenu 6 g de chlorhydrate de pyrazolo-[1,5-a]-pyrimidin-3-ylamine (solide rouge) après séchage sous vide et sur anhydride phosphorique (Rendement = 57,5%).
- RMN ¹H (D₂O ; 200MHz) :: 7,18 (dxd ; J₁ = 4,2 Hz et J₂ = 7,1 Hz; 1H) ; 8,33 (s ; 1H); 8,65 (dxd ; J₁ = 4,2 Hz et J₃ = 1,6 Hz ; 1H); 8,90 (dxd ; J₂ = 7,1 Hz et J₃ = 1,6 Hz ; 1H)

### EXEMPLE 2 : Synthèse du chlorhydrate de 5,6,7-triméthyl-pyrazolo-[1,5-a]-pyrimidin-3-ylamine

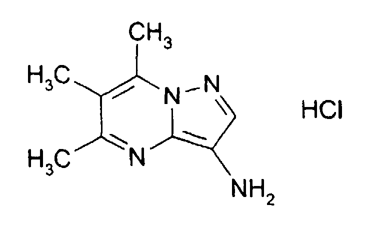

### a) Préparation de la 5,6,7-triméthyl-3-nitro-pyrazolo-[1,5-a]-pyrimidine

Dans un ballon tricol de 100 ml équipé d'une agitation magnétique, d'un réfrigérant et d'un thermomètre, on a introduit 80 cc d'acide acétique, 8,2 g de chlorhydrate de 4-nitro-2H-pyrazol-3-ylamine (préparé selon H. Dorn et H. Dilcher, Liebigs Ann.Chem.,707, 141, 1967), et 7,2 g de 3-méthyl pentanedione-2,4. On a porté le milieu au reflux pendant 3 heures. On a filtré le milieu à température ambiante et on a rincé le solide à l'éther diisopropylique. On a obtenu 9,4 g de produit brut. On en recristallise 3,5 g dans 23 cc d'éthanol absolu. On a obtenu 3,1 g de 5,6,7-triméthyl-3-nitro-pyrazolo-[1,5-a]-pyrimidine après séchage sous vide et sur anhydride phosphorique (Rendement = 80%).
- RMN 1H (DMSO d6; 200MHz) :: 2,31 (s ; 3H) ; 2,62 (s ; 3H) ; 2,75 (s ; 3H) ; 8,89 (s; 1H)

### b) Préparation du chlorhydrate de 5,6,7-triméthyl pyrazolo-[1,5-a]-pyrimidin-_{3-ylamine}

Dans un ballon tricol de 250 ml équipé d'une agitation magnétique, d'un réfrigérant et d'un thermomètre, on a introduit 90 cc d'éthanol, 10 cc d'eau, 1,5 g de chlorure d'ammonium et 6,18 g de 5,6,7-triméthyl-3-nitro-pyrazolo-[1,5-a]-pyrimidine obtenue ci-dessus à l'étape précédente. On a porté le milieu au reflux. On a retiré le chauffage et ajouté 7,8 g de zinc en poudre par petites portions de façon à maintenir le reflux. Après complète addition, on a chauffé pendant 1 heure au reflux. On a filtré à chaud les boues zinciques. On a concentré au dixième du volume et filtré le produit qui a cristallisé. On l'a rincé à l'éther de pétrole. On a obtenu 4,9 g de chlorhydrate de 5,6,7-triméthyl-pyrazolo-[1,5-a]-pyrimidin-3-ylamine après séchage sous vide et sur anhydride phosphorique (Rendement = 82%).
- RMN 1H (DMSO d6; 200MHz) :: 2,27 (s ; 3H) ; 2,55 (s ; 3H) ; 2,70 (s ; 3H) ; 8,22 (s ; 1H); 10,61 (s élargi ; 3H)

### EXEMPLE 3 : Synthèse du chlorhydrate de 7-méthyl-pyrazolo-[1,5-a]-pyrimidin-3-ylamine

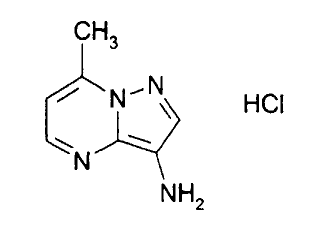

### a) Préparation de la 7-méthyl-3-nitro-pyrazolo-[1,5-a]-pyrimidine

Ce composé a été obtenu en suivant le protocole décrit à la première étape de l'exemple 2. Toutes les quantités ont été multipliées par quatre et la 3-méthyl pentanedione-2,4 a été remplacée par 1,1 équivalent molaire d'acétylacétaldehyde diméthylacétal. En fin de réaction, on a concentré la solution verte et on l'a versée sur de la glace. On a filtré le solide verdâtre qui a précipité, rincé à l'éther diisopropylique et à l'éther de pétrole. On a obtenu 26 g de 7-méthyl-3-nitro-pyrazolo-[1,5-a]-pyrimidine brut après séchage sous vide et sur anhydride phosphorique à 40°C (Rendement = 73%).
- RMN ¹H (DMSO d6; 200MHz): 2,82 (s ; 3H) ; 7,46 (d ; J = 4,5 Hz ; 1H) ; 8,90 (d ; J = 4,5 Hz ; 1H) ; 9,09 (s; 1H)

### b) Préparation du chlorhydrate de 7-méthyl pyrazolo-[1,5-a]-pyrimidin-3-ylamine

Dans un hydrogénateur de 1 litre, on a introduit 19,3 g de 7-méthyl-3-nitro-pyrazolo-[1,5-a]-pyrimidine, 500 cc de méthanol et 2,2 g de palladium sur charbon à 5% humide à 50%. On a chauffé le milieu vers 60°C et introduit 7,7 bars d'hydrogène. Après complète réduction, on a refroidi le réacteur et on a filtré le catalyseur. On a fait passer un courant d'acide chlorhydrique gazeux à travers le filtrat et filtré le chlorhydrate qui a précipité. On a obtenu 14,6 g de chlorhydrate de 7-méthyl-pyrazolo-[1,5-a]-pyrimidin-3-ylamine après séchage sous vide et sur anhydride phosphorique (Rendement = 72%).
- RMN ¹H (DMSO d6; 200MHz) :: 2,75 (s ; 3H) ; 7,17 (d ; J = 3,4 Hz ; 1H); 8,40 (s; 1H) ; 8,75 (d ; J = 4,2 Hz ; 1H) ; 10,77 (s élargi ; 3H)

### EXEMPLE 4 : Synthèse du chlorhydrate de 2,5,6,7-tetraméthyl-pyrazolo-[1,5-a]-pyrimidin-3-ylamine

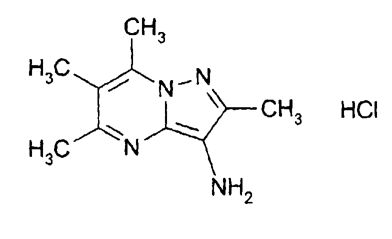

### a) Préparation de la 2,5,6,7-tetraméthyl-pyrazolo-[1,5-a]-pyrimidine

Dans un ballon tricol de 100 ml équipé d'une agitation magnétique, d'un réfrigérant et d'un thermomètre, on a introduit 12,6 g de 3-méthyl-2,4-pentanedione, 30 cc d'acide acétique et 9,6 g de 3-amino-5-méthyl-pyrazole. On a porté le milieu au reflux pendant 1 heure. On a évaporé l'acide acétique et on repris le produit dans 50 cc d'éther de pétrole. On a filtré le solide et on l'a rincé à l'éther de pétrole. On a obtenu 15 g de 2,5,6,7-tetra-méthyl-pyrazolo-[1,5-a]-pyrimidine après séchage sous vide et sur anhydride phosphorique (Rendement = 85,7%).
- RMN ¹H (DMSO d6; 200MHz) :: 2,06 (s ; 3H) ; 2,25 (s ; 3H) ; 2,31 (s ; 3H) ; 2,50 (s;3H); 6,13(s; 1H)

### b) Préparation de la 2,5,6,7-tetraméthyl-3-nitro-pyrazolo-[1,5-a]-pyrimidine

Dans un ballon tricol de 100 ml équipé d'une agitation magnétique, d'un réfrigérant, d'une ampoule à addition et d'un thermomètre, on a introduit 50 cc d'acide sulfurique à 98% dans lesquels on a solubilisé 12,25 g de 2,5,6,7-tetraméthyl-pyrazolo-[1,5-a]-pyrimidine obtenue ci-dessus à l'étape précédente. On a additionné goutte à goutte vers 0°C 4,4 g d'acide nitrique à 100% dilués dans 2,5 cc d'acide sulfurique à 98%. En fin d'addition, on a maintenu l'agitation à 0°C pendant 1 heure, puis versé le milieu sur 200 g de glace. On a amené le pH à 8 à l'aide d'ammoniaque à 20%. On a filtré le précipité. On l'a lavé à l'eau et rincé à l'éthanol et à l'éther diisopropylique. On a obtenu 11 g de 2,5,6,7-tetraméthyl-3-nitro-pyrazolo-[1,5-a]-pyrimidine après séchage sous vide et sur anhydride phosphorique (Rendement = 71,4%).
- RMN ¹H (DMSO d6; 200MHz) :: 2,33 (s; 3H) ; 2,63 (s; 3H) ; 2,67 (s; 3H) ; 2,75 (s; 3H)

### c) Préparation du chlorhydrate de 2,5,6,7-tetraméthyl-pyrazolo-[1,5-a]-pyrimidin-3-ylamine

Dans un ballon tricol de 100 ml équipé d'une agitation magnétique, d'un réfrigérant et d'un thermomètre, on a introduit 2,2 g de 2,5,6,7-tetraméthyl-3-nitro-pyrazolo-[1,5-a]-pyrimidine, 40 cc d'éthanol et 1 g de chlorure d'ammonium. On a porté le milieu au reflux et additionné 2,5 g de zinc en poudre par petites portions de façon à maintenir le reflux. Après 0,5 heure de reflux, on a filtré les boues zinciques. Le filtrat a été concentré jusqu'à cristallisation. On a filtré les cristaux. On a obtenu 2 g de chlorhydrate de 2,5,6,7-tetraméthyl-pyrazolo-[1,5-a]-pyrimidin-3-ylamine après séchage sous vide et sur anhydride phosphorique (Rendement = 88,2%).
- RMN ¹H (DMSO d6 ; 200MHz) :: 2,24 (s ; 3H) ; 2,35 (s ; 3H) ; 2,49 (s ; 3H) ; 2,62 (s ; 3H) ;6,34 (s élargi ; 3H)

### EXEMPLE 5 : Synthèse du chlorhydrate de 5,7-di-tert-butyl-pyrazolo-[1,5-a]-pyrimidin-3-ylamine

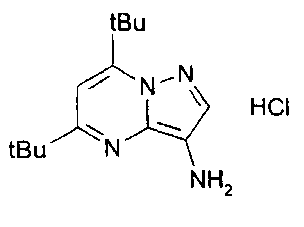

### a) Préparation de la 5,7-di-tert-butyl-3-nitro-pyrazolo-[1,5-a]-pyrimidine

Dans un tricol de 500 ml équipé d'un réfrigérant, d'un thermomètre et d'un barreau magnétique, on a introduit 36,85 g de 2,2,6,6-tétraméthyl-3,5-heptanedione, 200 cc d'acide acétique et 32,91 g de chlorhydrate de 4-nitro-2H-pyrazol-3-ylamine (préparé selon H. Dorn et H. Dilcher, Liebigs Ann.Chem.,707, 141, 1967). On a porté le mélange au reflux pendant 8,5 heures. La solution a été versée à chaud sur de la glace. On a filtré le solide jaune qui a précipité. On l'a recristallisé dans un mélange acide acétiqueleau. On a obtenu 25,4 g de 5,7-di-tert-butyl-3-nitro-pyrazolo-[1,5-a]-pyrimidine après séchage sous vide et sur anhydride phosphorique. (Rendement = 46%).
- RMN ¹H (DMSO d6; 200MHz) :: 1,40 (s; 9H) ; 1,57 (s; 9H) ; 7,26 (s; 1H); 8,98 (s; 1H);

### b) Préparation du chlorhydrate de 5,7-di-tert-butyl-pyrazolo-[1,5-a]-pyrimidin-3-ylamine

Dans un hydrogénateur de 1 litre, on a introduit 8 g de 5,7-di-tert-butyl-3-nitro-pyrazolo-[1,5-a]-pyrimidine, 350 cc d'éthanol et 2 g de palladium sur charbon à 5% contenant 50% d'eau. On a porté le milieu à 65°C et introduit une pression de 10,6 bars d'hydrogène. Après 1,5 heure, on a filtré le catalyseur sur de l'éthanol chlorhydrique 5M. On a traité ce filtrat avec du noir végétal. Après filtration du noir de charbon et évaporation de l'éthanol, on a obtenu 4,5 g de chlorhydrate de 5,7-di-tert-butyl-pyrazolo-[1,5-a]-pyrimidin-3-ylamine après séchage sous vide et sur anhydride phosphorique. (Rendement = 54,9%)
- RMN ¹H (DMSO d6; 200MHz) :: 1,40 (s; 9H) ; 1,58 (s; 9H) ; 7,02 (s; 1H); 8,35 (s; 1H) ; 10,55 (s élargi; 3H)

### EXEMPLE 6 : Synthèse du chlorhydrate de 5,7-di-trifluorométhyl-pyrazolo-[1,5-a]-pyrimidin-3-ylamine

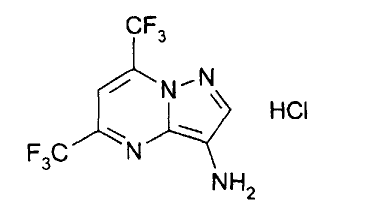

### a) Préparation de la 5,7-di-trifluorométhyl-3-nitro-pyrazolo-[1,5-a]-pyrimidine

Dans un tricol de 500 ml équipé d'un réfrigérant, d'un thermomètre et d'un barreau magnétique, on a introduit 31,2 g de 1,1,1,5,5,5-hexafluoro-2,4-pentanedione, 125 cc d'acide acétique et 24,7 g de chlorhydrate de 4-nitro-2H-pyrazol-3-ylamine (préparé selon H. Dorn et H. Dilcher, Liebigs Ann.Chem.,707, 141, 1967). On a porté le mélange au reflux pendant 8,5 heures. La solution a été versée à chaud sur de la glace. On a filtré le solide jaune qui a précipité. On l'a lavé à l'éther de pétrole. On a obtenu 31,9 g de 5,7-di-trifluorométhyl-3-nitro-pyrazolo-[1,5-a]-pyrimidine après séchage sous vide et sur anhydride phosphorique. (Rendement = 71%).
- RMN ¹H (DMSO d6; 200MHz) :: 8,67 (s; 1H); 9,54 (s; 1H)

### b) Préparation du chlorhydrate de 5,7-di-trifluorométhyl-pyrazolo-[1,5-a]-pyrimidin-3-ylamine

Dans un hydrogénateur de 250 cc, on a introduit 3 g de 5,7-di-trifluorométhyl-3-nitro-pyrazolo-[1,5-a]-pyrimidine, 100 cc d'éthanol et 0,4 g de palladium sur charbon à 5% contenant 50% d'eau. On a introduit une pression de 4,2 bars d'hydrogène. Après 1 heure et 40 minutes, on a filtré le catalyseur sur de l'éthanol chlorhydrique 5M. On a évaporé l'éthanol, et obtenu 2 g de chlorhydrate de 5,7-di-trifluorométhyl-pyrazolo-[1,5-a]-pyrimidin-3-ylamine brut. On en a recristallisé 1,6 g dans 7 cc d'éthanol chlorhydrique. On a obtenu 1 g de chlorhydrate de 5,7-di-trifluorométhyl-pyrazolo-[1,5-a]-pyrimidin-3-ylamine après séchage sous vide et sur anhydride phosphorique. (Rendement = 32,6%).
- RMN ¹H (DMSO d6; 200MHz) :: 8,23 (s; 1H) ; 8,72 (s; 1H) ; 9,54 (s élargi; 3H)

### EXEMPLES D'APPLICATION

### EXEMPLES 1 à 4 DE TEINTURE EN MILIEU ALCALIN

On a préparé les compositions tinctoriales, conformes à l'invention, suivantes (teneurs en grammes) :

| **COMPOSITION** | **1** | **2** | **3** | **4** |
|---|---|---|---|---|
| Chlorhydrate de pyrazolo-[1,5-a] | | | | |
| pyrimidine-3-ylamine (Base d'oxydation | 0,51 | - | - | - |
| de l'invention) | | | | |
| Chlorhydrate de 2,5,6,7-tetraméthyl | | | | |
| pyrazolo-[1,5-a] pyrimidine-3-ylamine | - | 0,68 | - | - |
| (Base d'oxydation de l'invention) | | | | |
| Chlorhydrate de 5,7-diméthyl pyrazolo- | | | | |
| [1,5-a] pyrimidine-3-ylamine (Base | - | - | 0,60 | - |
| d'oxydation de l'invention) | | | | |
| Chlorhydrate de 5,6,7-triméthyl pyrazolo- | | | | |
| [1,5-a] pyrimidine-3-ylamine (Base | - | - | - | 0,64 |
| d'oxydation de l'invention) | | | | |
| 2-méthyl-5-aminophénol (coupleur) | 0,37 | 0,37 | 0,37 | 0,37 |
| Support de teinture commun n°1 | (*) | (*) | (*) | (*) |
| Eau déminéralisée q.s.p. | 100 g | 100 g | 100 g | 100 g |

(*) Support de teinture commun n°1 :
- Alcool éthylique à 96° 18 g
- Métabisulfite de sodium en solution aqueuse à 35% 0,68 g
- Sel pentasodique de l'acide diéthylènetriaminopentacétique 1,1 g
- Ammoniaque à 20% 10,0 g
- Eau déminéralisée qs 100 g

Au moment de l'emploi, on a mélangé poids pour poids chacune des compositions tinctoriales ci-dessus avec une solution de peroxyde d'hydrogène à 20 volumes (6 % en poids) de pH 3.
Le mélange obtenu a été appliqué sur des mèches de cheveux gris naturels à 90 % de blancs pendant 30 minutes. Les mèches ont ensuite été rincées, lavées avec un shampooing standard, rincées à nouveau puis séchées.

Les nuances obtenues figurent dans le tableau ci-après :

| **EXEMPLE** | **Nuance obtenue** |
|---|---|
| **1** | Orangé cuivré |
| **2** | Orangé cuivré |
| **3** | Jaune orangé très léger |
| **4** | Jaune orangé léger |

### EXEMPLES 5 à 7 DE TEINTURE EN MILIEU ALCALIN

On a préparé les compositions tinctoriales, conformes à l'invention, suivantes (teneurs en grammes) :

| **COMPOSITION** | **5** | **6** | **7** |
|---|---|---|---|
| Chlorhydrate de 7-méthyl pyrazolo-[1,5-a] pyrimidine-3- | | | |
| ylamine (Base d'oxydation de l'invention) | 0,55 | - | - |
| Chlorhydrate de 5,7-di-tert-butyl pyrazolo- [1,5-a] | | | |
| pyrimidine-3-ylamine (Base d'oxydation de l'invention) | - | 0,85 | - |
| Chlorhydrate de 2-méthoxy 5,7-diméthyl pyrazolo-[1,5-a] | | | |
| pyrimidine-3-ylamine (Base d'oxydation de l'invention) | - | - | 0,69 |
| 2-méthyl-5-aminophénol (coupleur) | 0,37 | 0,37 | 0,37 |
| Support de teinture commun n°2 | (**) | (**) | (**) |
| Eau déminéralisée q.s.p. | 100 g | 100 g | 100 g |

(**) Support de teinture commun n°2 :
- Alcool éthylique à 96° 20 g
- Métabisulfite de sodium 0,23 g
- Agent séquestrant q.s.
- Ammoniaque à 20% 10,0 g
- Eau déminéralisée qs 100 g

Au moment de l'emploi, on a mélangé poids pour poids chacune des compositions tinctoriales ci-dessus avec une solution de peroxyde d'hydrogène à 20 volumes (6 % en poids) de pH 3.
Le mélange obtenu a été appliqué sur des mèches de cheveux gris naturels à 90 % de blancs pendant 30 minutes. Les mèches ont ensuite été rincées, lavées avec un shampooing standard, rincées à nouveau puis séchées.

Les nuances obtenues figurent dans le tableau ci-après :

| **EXEMPLE** | **Nuance obtenue** |
|---|---|
| **5** | Cuivré |
| **6** | Jaune |
| **7** | Doré cuivré |

### EXEMPLE 8 DE TEINTURE EN MILIEU ALCALIN

On a préparé la composition tinctoriale suivante :
- Chlorhydrate de 5,7-di-trifluorométhyl pyrazolo-[1,5-a]-pyrimidin-3-ylamine (Base d'oxydation de l'invention) 0,92 g
- 2-méthyl 5-aminophénol (Coupleur) 0,37 g
- Alcool benzylique 2 g
- Polyéthylèneglycol à 6 moles d'oxyde d'éthylène 3 g
- Ethanol à 96° 18 g
- Alkyl (C₈-C₁₀) polyglucoside en solution aqueuse à 60 % de matière active (M.A.) tamponné par du citrate d'ammonium vendu sous la dénomination ORAMIX CG110® par la Société SEPPIC 6 g
- Ammoniaque à 20 % de NH₃ 10 g
- Métabisulfite de sodium 0,23 g
- Agent séquestrant q.s.
- Eau déminéralisée q.s.p. 100 g

Au moment de l'emploi, on a mélangé poids pour poids la composition ci-dessus avec une solution de peroxyde d'hydrogène à 20 volumes (6 % en poids) de pH 3.

Le mélange obtenu a été appliqué sur des mèches de cheveux gris naturels à 90 % de blancs pendant 30 minutes. Les mèches ont ensuite été rincées, lavées avec un shampooing standard, rincées à nouveau puis séchées.

Les mèches de cheveux ont été teintes dans une nuance cuivré rouge.

## Revendications

1. Composition pour la teinture d'oxydation des fibres kératiniques, caractérisée par le fait qu'elle comprend, dans un milieu approprié pour la teinture, au moins un coupleur et au moins une 3-amino pyrazolo-[1,5-a]-pyrimidine à titre de base d'oxydation et/ou un de ses sels d'addition avec un acide ou avec une base :
choisie parmi :
- la pyrazolo-[1,5-a]-pyrimidin-3-ylamine ;
- la 5,6,7-triméthyl pyrazolo-[1,5-a]-pyrimidin-3-ylamine ;
- la 7-méthyl pyrazolo-[1,5-a]-pyrimidin-3-ylamine ;
- la 5-méthyl pyrazolo-[1,5-a]-pyrimidin-3-ylamine ;
- la 7-méthoxy pyrazolo-[1,5-a]-pyrimidin-3-ylamine ;
- la 5-méthoxy pyrazolo-[1,5-a]-pyrimidin-3-ylamine ;
- la 7-méthoxy 5-méthyl pyrazolo-[1,5-a]-pyrimidin-3-ylamine ;
- la 2,5,6,7-tetraméthyl pyrazolo-[1,5-a]-pyrimidin-3-ylamine ;
- la 2-méthoxy 5,7-diméthyl pyrazolo-[1,5-a]-pyrimidin-3-ylamine ;
- la 5,7-di-tert-butyl pyrazolo-[1,5-a]-pyrimidin-3-ylamine ;
- la 5,7-di-trifluorométhyl pyrazolo-[1,5-a]-pyrimidin-3-ylamine ;
- la 2,6-diméthyl pyrazolo-[1,5-a]-pyrimidin-3-ylamine ;
- la 2-chloro 5,7-diméthyl pyrazolo-[1,5-a]-pyrimidin-3-ylamine ;
- la 7-méthyl 2-tert-butyl 5-trifluorométhyl pyrazolo-[1,5-a]-pyrimidin-3-ylamine ;
et leurs sels d'addition avec un acide ou avec une base.

2. Composition selon la revendication 1, caractérisée par le fait que les 3-amino de pyrazolo-[1,5-a]-pyrimidines de formule (I) sont choisies parmi :
- la pyrazolo-[1,5-a]-pyrimidin-3-ylamine ;
- la 5,6,7-triméthyl pyrazolo-[1,5-a]-pyrimidin-3-ylamine ;
- la 2,5,6,7-tetraméthyl pyrazolo-[1,5-a]-pyrimidin-3-ylamine ;
- la 2-méthoxy 5,7-diméthyl pyrazolo-[1,5-a]-pyrimidin-3-ylamine ;
- la 5,7-di-trifluorométhyl pyrazolo-[1,5-a]-pyrimidin-3-ylamine ;
- la 7-méthyl pyrazolo-[1,5-a]-pyrimidin-3-ylamine ;
- la 5-méthyl pyrazolo-[1,5-a]-pyrimidin-3-ylamine ;
et leurs sels d'addition avec un acide ou avec une base.

3. Composition selon l'une quelconque des revendications 1 à 2, caractérisée par le fait que la ou les 3-amino pyrazolo-[1,5-a]-pyrimidines représentent de 0,0005 à 12 % en poids du poids total de la composition tinctoriale.

4. Composition selon la revendication 3, caractérisée par le fait que la ou les 3-amino pyrazolo-[1,5-a]-pyrimidines représentent de 0,005 à 6 % en poids du poids total de la composition tinctoriale.

5. Composition selon l'une quelconque des revendications précédentes, caractérisée par le fait que le milieu approprié pour la teinture (ou support) est constitué par de l'eau ou par un mélange d'eau et d'au moins un solvant organique choisi parmi les alcanols inférieurs en C₁-C₄, le glycérol, les glycols et éthers de glycols, les alcools aromatiques, et leurs mélanges.

6. Composition selon l'une quelconque des revendications précédentes, caractérisée par le fait qu'elle présente un pH compris entre 3 et 12.

7. Composition selon l'une quelconque des revendications précédentes, caractérisée par le fait que le ou les coupleurs sont choisis parmi les métaphénylènediamines, les méta-aminophénols, les métadiphénols et les coupleurs hétérocycliques.

8. Composition selon la revendication 7,caractérisée par le fait que les coupleurs sont choisis parmi le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxy benzène, le 1,3-diamino benzène, le 1,3-bis-(2,4-diaminophénoxy) propane, le sésamol, l'α-naphtol, le 6-hydroxy indole, le 4-hydroxy indole, le 4-hydroxy N-méthyl indole, la 6-hydroxy indoline, la 2,6-dihydroxy 4-méthyl pyridine, le 1-H 3-méthyl pyrazole 5-one, le 1-phényl 3-méthyl pyrazole 5-one, et leurs sels d'addition.

9. Composition selon la revendication 7 ou 8, caractérisée par le fait que le ou les coupleurs représentent de 0,0001 à 10 % en poids du poids total de la composition tinctoriale.

10. Composition selon la revendication 9, caractérisée par le fait que le ou les coupleurs représentent de 0,005 à 5 % en poids du poids total de la composition tinctoriale.

11. Composition selon l'une quelconque des revendications précédentes, caractérisée par le fait qu'elle renferme au moins une base d'oxydation additionnelle choisie parmi les paraphénylènediamines, les bis-phénylalkylènediamines, les para-aminophénols, les ortho-aminophénols et des bases hétérocycliques différentes des 3-amino pyrazolo-[1,5-a]-pyrimidines de formule (I).

12. Composition selon la revendication 11, caractérisée par le fait que la ou les bases d'oxydation additionnelles représentent de 0,0005 à 12 % en poids du poids total de la composition tinctoriale.

13. Composition selon la revendication 12, caractérisée par le fait que la ou les bases d'oxydation additionnelles représentent de 0,005 à 6 % en poids du poids total de la composition tinctoriale.

14. Composition selon l'une quelconque des revendications précédentes, caractérisée par le fait que les sels d'addition avec un acide sont choisis parmi les chlorhydrates, les bromhydrates, les sulfates, les citrates, les succinates, les tartrates, les lactates et les acétates, et que les sels d'addition avec une base sont choisis parmi ceux obtenus avec la soude, la potasse, l'ammoniaque ou les amines.

15. Procédé de teinture des fibres kératiniques caractérisé par le fait que l'on applique sur ces fibres au moins une composition tinctoriale telle que définie à l'une quelconque des revendications 1 à 14, pendant un temps suffisant pour développer la coloration désirée, soit à l'air, soit à l'aide d'un agent oxydant, éventuellement en présence de catalyseurs d'oxydation.

16. Procédé selon la revendication 15, caractérisé par le fait que l'on révèle la couleur à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

17. Procédé selon la revendication 15 ou 16, caractérisé par le fait que l'agent oxydant est choisi parmi le peroxyde d'hydrogène, le peroxyde d'urée, les bromates de métaux alcalins, les persels et les enzymes.

18. Dispositif à plusieurs compartiments, ou "kit" de teinture à plusieurs compartiments, dont un premier compartiment renferme une composition tinctoriale telle que définie à l'une quelconque des revendications 1 à 14 et un second compartiment renferme une composition oxydante.

19. 3-amino pyrazolo-[1,5-a]-pyrimidines choisies dans le groupe constitué par :
- la pyrazolo-[1,5-a]-pyrimidin-3-ylamine ;
- la 5,6,7-triméthyl pyrazolo-[1,5-a]-pyrimidin-3-ylamine ;
- la 7-méthyl pyrazolo-[1,5-a]-pyrimidin-3-ylamine ;
- la 5-méthyl pyrazolo-[1,5-a]-pyrimidin-3-ylamine ;
- la 7-méthoxy pyrazolo-[1,5-a]-pyrimidin-3-ylamine ;
- la 5-méthoxy pyrazolo-[1,5-a]-pyrimidin-3-ylamine ;
- la 7-méthoxy -5-methyl pyrazolo-[1,5-a]-pyrimidin-3-ylamine ;
- la 2,5,6,7-tetraméthyl pyrazolo-[1,5-a]-pyrimidin-3-ylamine ;
- la 5,7-di-tert-butyl pyrazolo-[1,5-a]-pyrimidin-3-ylamine ;
- la 5,7-di-trifluorométhyl pyrazolo-[1,5-a]-pyrimidin-3-ylamine ;
et leurs sels d'addition avec un acide ou avec une base.

## Claims

1. Composition for the oxidation dyeing of keratinous fibres, characterized in that it comprises, in a medium appropriate for dyeing, at least one coupler and at least one 3-aminopyrazolo[1,5a]pyrimidine, as oxidation base, and/or one of its addition salts with an acid or with a base, selected from:
- pyrazolo[1,5-a]pyrimidin-3-ylamine;
- 5,6,7-trimethylpyrazolo[1,5-a]pyrimidin-3-ylamine;
- 7-methylpyrazolo[1,5-a]pyrimidin-3-ylamine;
- 5-methylpyrazolo[1,5-a]pyrimidin-3-ylamine;
- 7-methoxypyrazolo[1,5-a]pyrimidin-3-ylamine;
- 5-methoxypyrazolo[1,5-a]pyrimidin-3-ylamine;
- 7-methoxy-5-methylpyrazolo[1,5-a]pyrimidin-3-ylamine;
- 2,5,6,7-tetramethylpyrazolo[1,5-a]pyrimidin-3-ylamine;
- 2-methoxy-5,7-dimethylpyrazolo[1,5-a]pyrimidin-3-ylamine;
- 5,7-di-tert-butylpyrazolo[1,5-a]pyrimidin-3-ylamine;
- 5,7-di-trifluoromethylpyrazolo[1,5-a]pyrimidin-3-ylamine;
- 2,6-dimethylpyrazolo[1,5-a]pyrimidin-3-ylamine;
- 2-chloro-5,7-dimethylpyrazolo[1,5-a]pyrimidin-3-ylamine;
- 7-methyl 2-tert-butyl-5-trifluoromethyl-pyrazolo-[1,5-a]pyrimidin-3-ylamine;
and their addition salts with an acid or with a base.

2. Composition according to Claim 1, characterized in that the 3-aminopyrazolo[1,5-a]-pyrimidines of formula (I) are selected from:
- pyrazolo[1,5-a]pyrimidin-3-ylamine;
- 5,6,7-trimethylpyrazolo[1,5-a]pyrimidin-3-ylamine;
- 2,5,6,7-tetramethylpyrazolo[1,5-a]pyrimidin-3-ylamine;
- 2-methoxy-5,7-dimethylpyrazolo[1,5-a]pyrimidin-3-ylamine;
- 5,7-di-trifluoromethylpyrazolo[1,5-a]pyrimidin-3-ylamine;
- 7-methylpyrazolo[1,5-a]pyrimidin-3-ylamine;
- 5-methylpyrazolo[1,5-a]pyrimidin-3-ylamine;
and their addition salts with an acid or with a base.

3. Composition according to any one of Claims 1 to 2, characterized in that the 3-amino-pyrazolo[1,5-a]pyrimidine or -pyrimidines makes or make up from 0.0005 to 12% by weight of the total weight of the dyeing composition.

4. Composition according to Claim 3, characterized in that the 3-aminopyrazolo[1,5-a]-pyrimidine or -pyrimidines makes or make up from 0.005 to 6% by weight of the total weight of the dyeing composition.

5. Composition according to any one of the preceding claims, characterized in that the medium appropriate for dyeing (or vehicle) consists of water or of a mixture of water and at least one organic solvent selected from C₁-C₄ lower alkanols, glycerol, glycols and glycol ethers, aromatic alcohols, and mixtures thereof.

6. Composition according to any one of the preceding claims, characterized in that it has a pH of between 3 and 12.

7. Composition according to any one of the preceding claims, characterized in that the coupler or couplers is or are selected from metaphenylenediamines, meta-aminophenols, meta-diphenols and heterocyclic couplers.

8. Composition according to Claim 7, characterized in that the couplers are selected from 2-methyl-5-aminophenol, 5-N-(β-hydroxyethyl)amino-2-methylphenol, 3-aminophenol, 1,3-dihydroxybenzene, 1,3-dihydroxy-2-methylbenzene, 4-chloro-1,3-dihydroxybenzene, 2,4-diamino-1-(β-hydroxyethyloxy)benzene, 2-amino-4-(β-hydroxyethylamino)-1-methoxybenzene, 1,3-diaminobenzene, 1,3-bis(2,4-diaminophenoxy)propane, sesamol, α-naphthol, 6-hydroxyindole, 4-hydroxyindole, 4-hydroxy-N-methylindole, 6-hydroxyindoline, 2,6-dihydroxy-4-methylpyridine, 1-H-3-methylpyrazol-5-one, 1-phenyl-3-methylpyrazol-5-one, and their addition salts.

9. Composition according to Claim 7 or 8, characterized in that the coupler or couplers makes or make up from 0.0001 to 10% by weight of the total weight of the dyeing composition.

10. Composition according to Claim 9, characterized in that the coupler or couplers makes or make up from 0.005 to 5% by weight of the total weight of the dyeing composition.

11. Composition according to any one of the preceding claims, characterized in that it includes at least one additional oxidation base selected from para-phenylenediamines, bisphenylalkylenediamines, para-aminophenols, ortho-aminophenols and heterocyclic bases which are different from the 3-aminopyrazolo[1,5-a]pyrimidines of formula (I).

12. Composition according to Claim 11, characterized in that the additional oxidation base or bases makes or make up from 0.0005 to 12% by weight of the total weight of the dyeing composition.

13. Composition according to Claim 12, characterized in that the additional oxidation base or bases makes or make up from 0.005 to 6% by weight of the total weight of the dyeing composition.

14. Composition according to any one of the preceding claims, characterized in that the addition salts with an acid are selected from hydrochlorides, hydrobromides, sulphates, citrates, succinates, tartrates, lactates and acetates and in that the addition salts with a base are selected from those obtained with sodium hydroxide, potassium hydroxide, aqueous ammonia, or amines.

15. Method of dyeing keratinous fibres, characterized in that at least one dyeing composition as defined in any one of Claims 1 to 14 is applied to these fibres for a time which is sufficient for the desired coloration to develop either in air or with the aid of an oxidizing agent, optionally in the presence of oxidation catalysts.

16. Method according to Claim 15, characterized in that the colour is developed at an acidic, neutral or alkaline pH with the aid of an oxidizing agent which is added right at the time when the dyeing composition is employed or which is present in an oxidizing composition which is applied simultaneously or sequentially and separately.

17. Method according to Claim 15 or 16, characterized in that the oxidizing agent is selected from hydrogen peroxide, urea peroxide, alkali metal bromates, persalts and enzymes.

18. Multi-compartment device or multi-compartment dyeing kit of which a first compartment contains a dyeing composition as defined in any one of Claims 1 to 14 and a second compartment contains an oxidizing composition.

19. 3-Aminopyrazolo[1,5-a]pyrimidines selected from the group consisting of:
- pyrazolo[1,5-a]pyrimidin-3-ylamine;
- 5,6,7-trimethylpyrazolo[1,5-a]pyrimidin-3-ylamine;
- 7-methylpyrazolo[1,5-a]pyrimidin-3-ylamine;
- 5-methylpyrazolo[1,5-a]pyrimidin-3-ylamine;
- 7-methoxypyrazolo[1,5-a]pyrimidin-3-ylamine;
- 5-methoxypyrazolo[1,5-a]pyrimidin-3-ylamine;
- 7-methoxy-5-methylpyrazolo[1,5-a]pyrimidin-3-ylamine;
- 2,5,6,7-tetramethylpyrazolo[1,5-a]pyrimidin-3-ylamine;
- 5,7-di-tert-butylpyrazolo[1,5-a]pyrimidin-3-ylamine;
- 5,7-di-trifluoromethylpyrazolo[1,5-a]pyrimidin-3-ylamine;
and their addition salts with an acid or with a base.

## Patentansprüche

1. Zusammensetzung zum oxidativen Färben von Keratinfasern und insbesondere menschlichen Keratinfasern, wie dem Haar, dadurch gekennzeichnet, daß sie in einem zum Färben geeigneten Medium mindestens einen Kuppler und als Oxidationsbase mindestens ein 3-Amino-pyrazolo-[1,5-a]-pyrimidin oder ein Additionssalz dieser Verbindungen mit einer Säure oder einer Base enthält, das ausgewählt ist unter:
- Pyrazolo-[1,5-a]-pyrimidin-3-ylamin;
- 5,6,7-Trimethyl-pyrazolo-[1,5-a]-pyrimidin-3-ylamin;
- 7-Methyl-pyrazolo-[1,5-a]-pyrimidin-3-ylamin;
- 5-Methyl-pyrazolo-[1,5-a]-pyrimidin-3-ylamin;
- 7-Methoxy-pyrazolo-[1,5-a]-pyrimidin-3-ylamin;
- 5-Methoxy-pyrazolo-[1,5-a]-pyrimidin-3-ylamin;
- 7-Methoxy-5-methyl-pyrazolo-[1,5-a]-pyrimidin-3-ylamin;
- 2,5,6,7-Tetramethyl-pyrazolo-[1,5-a]-pyrimidin-3-ylamin;
- 2-Methoxy-5,7-dimethyl-pyrazolo-[1,5-a]-pyrimidin-3-ylamin;
- 5,7-Di-*tert*.-butyl-pyrazolo-[1,5-a]-pyrimidin-3-ylamin;
- 5,7-Di-trifluormethyl-pyrazolo-[1,5-a]-pyrimidin-3-ylamin;
- 2,6-Dimethyl-pyrazolo-[1,5-a]-pyrimidin-3-ylamin;
- 2-Chlor-5,7-dimethyl-pyrazolo-[1,5-a]-pyrimidin-3-ylamin;
- 7-Methyl-2-*tert.*-butyl-5-trifluormethyl-pyrazolo-[1,5-a]-pyrimidin-3-ylamin; und
- deren Additionssalze mit einer Säure oder Base.

2. Zusammensetzung nach Anspruch 1, dadurch gekennzeichnet, daß die 3-Amino-pyrazolo-[1,5-a]-pyrimidine ausgewählt sind unter:
- Pyrazolo-[1,5-a]-pyrimidin-3-ylamin;
- 5,6,7-Trimethyl-pyrazolo-[1,5-a]-pyrimidin-3-ylamin;
- 2,5,6,7-Tetramethyl-pyrazolo-[1,5-a]-pyrimidin-3-ylamin;
- 2-Methoxy-5,7-dimethyl-pyrazolo-[1,5-a]-pyrimidin-3-ylamin;
- 5,7-Di-trifluormethyl-pyrazolo-[1,5-a]-pyrimidin-3-ylamin;
- 7-Methyl-pyrazolo-[1,5-a]-pyrimidin-3-ylamin;
- 5-Methyl-pyrazolo-[1,5-a]-pyrimidin-3-ylamin; und
- deren Additionssalze mit einer Säure oder Base.

3. Zusammensetzung nach einem der Ansprüche 1 oder 2, dadurch gekennzeichnet, daß das oder die 3-Amino-pyrazolo-[1,5-a]-pyrimidine 0,0005 bis 12 Gew.-% des Gesamtgewichts der Färbemittelzusammensetzung ausmachen.

4. Zusammensetzung nach Anspruch 3, dadurch gekennzeichnet, daß das oder die 3-Amino-pyrazolo-[1,5-a]-pyrimidine 0,005 bis 6 Gew.-% des Gesamtgewichts der Färbemittelzusammensetzung ausmachen.

5. Zusammensetzung nach einem der vorhergehenden Ansprüche, dadurch gekennzeichnet, daß das zum Färben geeignete Medium (oder der Träger) aus Wasser oder einem Gemisch von Wasser und mindestens einem organischen Lösungsmittel besteht, das unter den niederen C₁₋₄-Alkanolen, Glycerin, Glykolen und Glykolethern, aromatischen Alkoholen und deren Gemischen ausgewählt ist.

6. Zusammensetzung nach einem der vorhergehenden Ansprüche, dadurch gekennzeichnet, daß sie einen pH-Wert von 3 bis 12 aufweist.

7. Zusammensetzung nach einem der vorhergehenden Ansprüche, dadurch gekennzeichnet, daß der oder die Kuppler unter den m-Phenylendiaminen, m-Aminophenolen, m-Dihydroxybenzolen und den heterocyclischen Kupplern ausgewählt sind.

8. Zusammensetzung nach Anspruch 7, dadurch gekennzeichnet, daß die Kuppler unter 2-Methyl-5-amino-phenol, 5-N-(β-Hydroxyethyl)amino-2-methyl-phenol, 3-Amino-phenol, 1,3-Dihydroxy-benzol, 1,3-Dihydroxy-2-methyl-benzol, 4-Chlor-1,3-dihydroxy-benzol, 2,4-Diamino-1-(ß-hydroxyethyloxy)-benzol, 2-Amino-4-(β-hydroxyethylamino)-1-methoxy-benzol, 1,3-Diamino-benzol, 1,3-Bis(2,4-diaminophenoxy)-propan, Sesamol, α-Naphthol, 6-Hydroxyindol, 4-Hydroxyindol, 4-Hydroxy-N-methyl-indol, 6-Hydroxyindolin, 2,6-Dihydroxy-4-methyl-pyridin, 1-H-3-Methyl-pyrazol-5-on, 1-Phenyl-3-methyl-pyrazol-5-on und den Additionssalzen dieser Verbindungen ausgewählt sind.

9. Zusammensetzung nach Anspruch 7 oder 8, dadurch gekennzeichnet, daß der oder die Kuppler 0,0001 bis 10 Gew.-% des Gesamtgewichts der Färbemittelzusammensetzung ausmachen.

10. Zusammensetzung nach Anspruch 9, dadurch gekennzeichnet, daß der oder die Kuppler 0,005 bis 5 Gew.-% des Gesamtgewichts der Färbemittelzusammensetzung ausmachen.

11. Zusammensetzung nach einem der vorhergehenden Ansprüche, dadurch gekennzeichnet, daß sie mindestens eine zusätzliche Oxidationsbase enthält, die unter den p-Phenylendiaminen, Bisphenylalkylendiaminen, p-Aminophenolen, o-Amino-phenolen und heterocyclischen Basen, die von den 3-Amino-pyrazolo-[1,5-a]-pyrimidinen verschieden sind, ausgewählt sind.

12. Zusammensetzung nach Anspruch 11, dadurch gekennzeichnet, daß die zusätzliche(n) Oxidationsbase(n) 0,0005 bis 12 Gew.-% des Gesamtgewichts der Färbemittelzusammensetzung ausmachen.

13. Zusammensetzung nach Anspruch 12, dadurch gekennzeichnet, daß die zusätzliche(n) Oxidationsbase(n) 0,005 bis 6 Gew.-% des Gesamtgewichts der Färbemittelzusammensetzung ausmachen.

14. Zusammensetzung nach einem der vorhergehenden Ansprüche, dadurch gekennzeichnet, daß die Additionssalze mit einer Säure unter den Hydrochloriden, Hydrobromiden, Sulfaten, Citraten, Succinaten, Tartraten, Lactaten und Acetaten und die Additionsalze mit einer Base unter den Salzen ausgewählt sind, die mit Natriumhydroxid, Kaliumhydroxid, Ammoniak oder Aminen erhalten werden.

15. Verfahren zum Färben von Keratinfasern, dadurch gekennzeichnet, daß mindestens eine Färbemittelzusammensetzung nach einem der Ansprüche 1 bis 14 während einer Zeitspanne auf die Faser aufgebracht wird, die ausreichend ist, um die gewünschte Färbung gegebenenfalls in Gegenwart von Oxidationskatalysatoren entweder mit Luftsauerstoff oder einem Oxidationsmittel zu entwickeln.

16. Verfahren nach Anspruch 15, dadurch gekennzeichnet, daß die Farbe bei einem sauren, neutralen oder alkalischen pH-Wert mit einem Oxidationsmittel entwickelt wird, das bei der Anwendung zu der Färbemittelzusammensetzung gegeben wird oder das in einer oxidierenden Zusammensetzung vorliegt, die gleichzeitig oder getrennt davon anschließend aufgetragen wird.

17. Verfahren nach Anspruch 15 oder 16, dadurch gekennzeichnet, daß das Oxidationsmittel unter Wasserstoffperoxid, Harnstoffperoxid, Alkalimetallbromaten, Salzen von Persäuren und Enzymen ausgewählt ist.

18. Vorrichtung mit mehreren Abteilungen oder "Kit" zum Färben mit mehreren Abteilungen, wobei eine erste Abteilung eine Färbemittelzusammensetzung nach einem der Ansprüche 1 bis 14 und eine zweite Abteilung eine oxidierende Zusammensetzung enthält.

19. 3-Amino-pyrazolo-[1,5-a]-pyrimidine, die ausgewählt sind unter:
- Pyrazolo-[1,5-a]-pyrimidin-3-ylamin;
- 5,6,7-Trimethyl-pyrazolo-[1,5-a]-pyrimidin-3-ylamin;
- 7-Methyl-pyrazolo-[1,5-a]-pyrimidin-3-ylamin;
- 5-Methyl-pyrazolo-[1,5-a]-pyrimidin-3-ylamin;
- 7-Methoxy-pyrazolo-[1,5-a]-pyrimidin-3-ylamin;
- 5-Methoxy-pyrazolo-[1,5-a]-pyrimidin-3-ylamin;
- 7-Methoxy-5-methyl-pyrazolo-[1,5-a]-pyrimidin-3-ylamin;
- 2,5,6,7-Tetramethyl-pyrazolo-[1,5-a]-pyrimidin-3-ylamin;
- 5,7-Di-*tert.*-butyl-pyrazolo-[1,5-a]-pyrimidin-3-ylamin;
- 5,7-Di-trifluormethyl-pyrazolo-[1,5-a]-pyrimidin-3-ylamin;und
- deren Additionssalzen mit einer Säure oder Base.
